# FASCICULE DE BREVET EUROPEEN

(11) **EP 1 819 395 B1**
(45) Date de publication et mention de la délivrance du brevet: **06.04.2016**
(21) Numéro de dépôt: 05826521.6
(22) Date de dépôt: 09.12.2005
(51) Int. Cl.: A61N 1/32, A61N 1/34, A61N 1/36

(54) **SYSTEME D'ELECTRODES POUR STIMULATION TRANSCUTANEE DE NERFS ET/OU DE MUSCLES**
ELEKTRODENSYSTEM FÜR TRANSKUTANE NERVEN- UND/ODER MUSKELSTIMULATION
ELECTRODE SYSTEM FOR TRANSCUTANEOUS NERVE AND/OR MUSCLE STIMULATION

(30) Priorité: 09.12.2004 CH 204404
(43) Date de publication de la demande: 22.08.2007
(73) Titulaire: COMPEX MEDICAL S.A., 1024 Ecublens (CH)
(72) Inventeur: BUHLMANN, Félix, 1018 Lausanne (CH); SCHÖNENBERGER, Klaus, 1031 Mex (CH); RIGAUX, Pierre, 4537 Verlaine (BE)
(74) Mandataire: Grosfillier, Philippe
(86) Numéro de dépôt international: PCT/IB2005/054156
(87) Numéro de publication internationale: WO 2006/061805

(56) Documents cités:
- EP-A- 0 830 875
- WO-A1-91/19535
- US-A- 5 052 391
- US-A- 5 111 812
- US-B1- 6 341 237
- US-B1- 6 438 418

## Description

### Domaine de l'invention

La présente invention concerne la stimulation électrique transcutanée de nerfs et de muscles au moyen d'un système comprenant une paire d'électrodes reliées à un générateur de courant.

Ce type de stimulation peut être utilisé dans un grand nombre de domaines. A titre non exhaustif, on peut citer les domaines de l'électrostimulation neuro-musclaire à des fins sportives ou médicales, de l'anesthésie, de la défibrillation ou de la régulation des battements cardiaques au moyen d'un pace maker externe.

### Etat de la technique

Pour stimuler de manière transcutanée des nerfs et/ou des muscles, il est connu de générer un courant entre deux électrodes disposées sur la peau. A titre d'exemple illustratif, les demandes WO 91/19535 et EP 1 095 670 décrivent un tel stimulateur électrique neuromusculaire utilisant des électrodes de stimulation et un générateur d'impulsions électriques. Outre les nerfs et/ou les muscles, le courant stimule également les récepteurs sous-cutanés qui sont disposés entre la surface de la peau et les nerfs et/ou les muscles à stimuler.

Toutefois, les récepteurs sous-cutanés sont sensibles à divers facteurs tels que la température, la pression ou la douleur. En conséquence, leur excitation par un courant électrique peut induire des effets indésirables pour l'utilisateur tels que douleur, picotements, etc...

Il existe donc un besoin de pouvoir réduire, voire éliminer les sensations indésirables ressenties lors de la stimulation transcutanée de nerfs et/ou de muscles par des électrodes.

### Résumé de l'invention

Ainsi, un but de l'invention est d'améliorer les systèmes connus dans l'état de la technique.

Plus particulièrement, un but de l'invention est de proposer un système de stimulation électrique transcutanée des nerfs et des muscles qui remédie au problème précité de l'apparition de sensations indésirables lors de la stimulation.

Un autre but de l'invention est de proposer un dispositif simple et facile à mettre en oeuvre qui soit efficace et apporte un meilleur confort à l'utilisateur.

A cet effet, l'invention porte sur un système d'électrodes pour stimulation transcutanée de nerfs et/ou de muscles selon la revendication 1.

Par "proximité" on entend une distance plus courte que la distance entre les électrodes de stimulation.

Ainsi, selon l'invention, le système comprend deux circuits, l'un pour stimuler les muscles et/ou les nerfs et l'autre pour rendre les récepteurs sous-cutanés moins excitables augmentant en cela le confort de l'utilisateur. Cette action spécifique sur les récepteurs sous-cutanés est rendue possible par la présence d'électrodes qui sont proches les unes des autres, de sorte que le courant injecté ne va pas en profondeur mais reste en surface. De plus, les formes de courant injecté sont choisies de façon à atteindre ce but.

Avantageusement l'électrode d'injection de courant est disposée autour de l'électrode de stimulation.

Selon un mode de réalisation particulier de l'invention, chaque électrode de stimulation est entourée par une électrode d'injection de courant.

Selon un autre mode de réalisation particulier, l'électrode de stimulation comprend une première partie entourée par une première partie d'électrode d'injection de courant, elle-même étant entourée d'une deuxième partie de l'électrode de stimulation qui elle-même est entourée d'une deuxième partie de l'électrode d'injection de courant . Cette configuration peut se répéter plusieurs fois.

Selon un exemple une méthode d'utilisation du système d'électrodes est divulguée qui se caractérisé par le fait que l'on choisit une forme de courant générée par les moyens de génération de courant au niveau de ladite électrode d'injection de manière à réduire, voire à éliminer, les sensations indésirables pouvant résulter de l'excitation des récepteurs sous-cutanés par ledit courant de stimulation.

Une première façon d'atteindre cet objectif est de choisir une intensité de courant d'injection de manière a diminuer ou à annuler le courant total circulant au niveau des récepteurs sous-cutanés. Dans ce cas, le courant injecté, qui se dirige vers l'électrode de stimulation correspondante, compense le courant de stimulation qui sort de l'électrode de stimulation.

On notera ici que la compensation courant de stimulation - courant injecté résulte en une annulation du courant total exclusivement au niveau des récepteurs sous-cutanés. Une telle annulation de courant ne se produit pas au niveau des nerfs et/ou muscles à stimuler.

Une deuxième façon de procéder est de choisir une forme de courant d'injection de manière à induire un blocage de la transmission nerveuse vers les récepteurs sous-cutanés. On obtient de la sorte un effet anesthésiant sur les récepteurs sous-cutanés.

Une troisième façon consiste à exciter, sans induire de douleur, l'environnement des récepteurs sous-cutanés (effet TENS). Dans ce cas, le signal de douleur envoyé par les récepteurs vers le cerveau est noyé dans un signal général. Typiquement, dans l'effet TENS, un fourmillement permanent est perçu par l'utilisateur, fourmillement qui masque d'autres sensations telles que la douleur. De cette façon, on couvre une sensation par une autre.

Une autre façon de procéder consiste à envoyer d'abord un courant local qui rend les récepteurs sous-cutanés moins excitables puis à envoyer le courant de stimulation.

On peut utiliser différents courants décalés dans le temps.

Selon une variante, on charge les récepteurs et ceux-ci seront moins sensibles au courant de stimulation.

Typiquement, on peut appliquer les principes exposés dans les publications WO 02/065896 ou US 2004/0127953 pour les formes de courants générés dans le système selon l'invention.

D'autres exemples sont donnés dans les publications suivantes:
-) Bhadra N, Kilgore KL, "Direct current electrical conduction block of peripheral nerve", IEEE Trans Neural Syst Rehabil Eng. 2004 Sep;12(3):313-24;
-) Kilgore KL, Bhadra N. "Nerve conduction block utilising high-frequency alternating current", Med Biol Eng Comput. 2004 May;42(3):394-406;
-) Bhadra N, Kilgore KL, Creasey GH, "Block of Mammalian Motor Nerve Conduction Using High Frequency Alternating Current", 10th Annual Conference of the International FES Society, July 2005, Montreal, Canada;
-) Kilgore KL, Bhadra N, "Block of Nerve Conduction Using High Frequency Alternating Current", 9th Annual Conférence of the International FES Society, September 2004, Bournemouth, UK.

Dans ces documents, on trouve des explications sur les différentes méthodes qui permettent d'obtenir un bloc de conduction (bloc de collision, hyperpolarisation, dépolarisation, bloc AC haute fréquence).

Comme indiqué ci-dessus, on peut également utiliser des formes de courants qui soit masquent les sensations de douleur (TENS) soit diminuent la densité de courant ou la densité de charge électrique au niveau des fibres sensitives de la peau. Cette liste est bien entendu non-exhaustive et d'autres formes sont envisageables, l'idée étant d'envoyer un courant d'injection sur les récepteurs sous-cutanés pour les rendre moins excitables.

### Brève description des figures

L'invention sera mieux comprise dans la description détaillée qui suit de deux modes d'exécution de celle-ci. A cet effet, un exemple non-limitatif est schématisé au moyen des figures suivantes :
La figure 1 illustre une coupe du système selon l'invention disposé sur la peau.
La figure 2 représente une vue de dessus du système de la figure 1.
La figure 3 illustre un autre mode d'exécution de l'invention.

### Description détaillée des modes de réalisation de l'invention

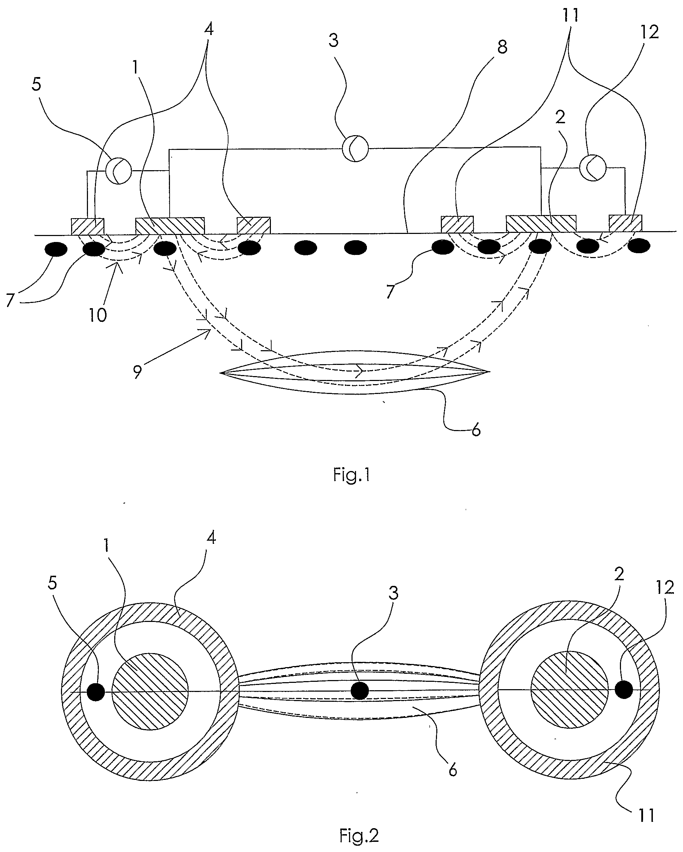

Le système selon le premier mode d'exécution illustré sur les figures 1 et 2 comprend deux électrodes de stimulation **1,2** reliées à un générateur de courant de stimulation **3**. Sous la peau **8**, un courant **9** de stimulation se déplace entre les électrodes de stimulation **1,2** et stimule un muscle **6**.

Chaque électrode de stimulation **1,2** est entourée d'une électrode d'injection de courant **4**, respectivement **11** de forme annulaire, dans l'exemple représenté. Les électrodes d'injection de courant **4,11** sont reliées aux électrodes de stimulation **1,2** par un générateur de courant d'injection **5**, respectivement **1**2.

Suivant la disposition des électrodes, de leur géométrie et des formes de courants choisis, on obtient l'effet recherché par exemple une annulation partielle, voire totale du courant se déplaçant au niveau des capteurs sous-cutanés **7**, un blocage au niveau de la transmission nerveuse entre les récepteurs et le cerveau (effet anesthésiant) ou une excitation de l'environnement dans lequel se trouvent les récepteurs (effet TENS).

Les sensations indésirables induites par une excitation parasite des récepteurs sous-cutanés **7** sont donc réduites, ou masquées, voire totalement éliminées.

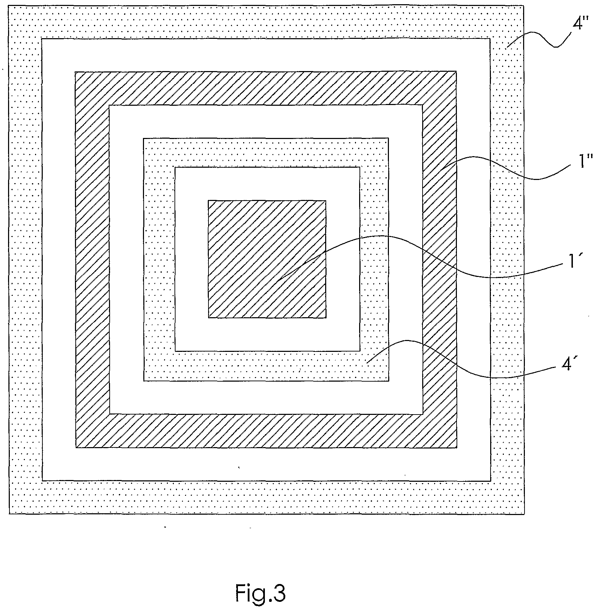

Dans le mode d'exécution illustré à la figure 3, l'électrode de stimulation comprend une première partie **1'** entourée par une première partie d'électrode d'injection de courant **4'**, elle-même étant entourée d'une deuxième partie de l'électrode de stimulation **1"** qui elle-même est entourée d'une deuxième partie de l'électrode d'injection de courant **4"**. Cette configuration peut bien entendu se répéter.

Il va de soi que l'invention ne se limite pas aux exemples précités.

De manière générale, elle concerne toute configuration d'électrodes telle que décrite plus haut qui permet de réduire ou éliminer les sensations indésirables résultant d'une excitation des récepteurs sous-cutanés. En particulier, plus on veut rester en surface au niveau des récepteurs sous-cutanés avec le courant d'injection, plus les électrodes d'injection et de stimulation doivent être proches les unes des autres.

### Liste des références numériques

- 1.: Première électrode de stimulation
- 2.: Deuxième électrode de stimulation
- 3.: Générateur de courant de stimulation
- 4.: Première électrode d'injection de courant
- 5.: Premier générateur d'injection de courant
- 6.: Muscle
- 7.: Récepteur sous-cutané
- 8.: Surface de la peau
- 9.: Courant de stimulation
- 10.: Courant de compensation
- 11.: Deuxième électrode d'injection de courant
- 12.: Deuxième générateur d'injection de courant
- 1': Première partie de l'électrode de simulation (2^{e} mode)
- 1": Deuxième partie de l'électrode de simulation (2^{e} mode)
- 4': Première partie de l'électrode d'injection (2^{e} mode)
- 4": Deuxième partie de l'électrode d'injection (2^{e} mode)

## Revendications

1. Système d'électrodes pour stimulation transcutanée de nerfs et/ou de muscles comprenant :
- une paire d'électrodes de stimulation (1,2) ayant une distance entre elles,
- un générateur de courant (3) relié aux électrodes de stimulation pour générer un courant (9) de stimulation de nerfs et/ou de muscles entre lesdites électrodes de stimulation (1,2),
- une paire d'électrodes d'injection de courant (4,11) disposées à une distance de l'une des électrodes de stimulation qui est inférieure à la distance entre la paire d'électrodes de stimulation (1,2)
- un générateur de courant d'injection (5,12) relié à une d'électrodes de stimulation (1,2) et à une d'électrodes d'injection de courant,
l'électrode d'injection de courant (4,11) et le générateur de courant correspondant (5,12) étant configuré pour produire un courant injecté pour réduire, voire éliminer, des sensations indésirables résultant de l'excitation par ledit courant de stimulation, **caractérisé en ce que** chaque électrode de stimulation (1,2) est entourée par une électrode d'injection de courant (4,11) et **en ce qu'**une électrode de stimulation comprend au moins une première partie (1') complètement entourée par une première partie d'électrode d'injection de courant (4'), elle-même étant complètement entourée d'une deuxième partie de l'électrode de stimulation (1 ") qui elle-même est complètement entourée d'une deuxième partie de l'électrode d'injection de courant (4").

2. Système selon la revendication 1, **caractérisé en ce que** la configuration des électrodes se répète.

## Patentansprüche

1. Elektrodensystem zur transkutanen Stimulation von Nerven und/oder Muskeln, das enthält:
- ein Paar von Stimulationselektroden (1, 2), die einen Abstand zueinander haben,
- einen Stromgenerator (3), der mit den Stimulationselektroden verbunden ist, um einen Strom (9) zur Stimulation von Nerven und/oder Muskeln zwischen den Stimulationselektroden (1, 2) zu erzeugen,
- ein Paar von Stromeinspeiseelektroden (4, 11), die in einem Abstand zu einer der Stimulationselektroden angeordnet sind, der kleiner ist als der Abstand zwischen dem Paar von Stimulationselektroden (1, 2)
- einen Einspeisestromgenerator (5, 12), der mit einer der Stimulationselektroden (1, 2) und mit einer der Stromeinspeiseelektroden verbunden ist,
wobei die Stromeinspeiseelektrode (4, 11) und der entsprechende Stromgenerator (5, 12) konfiguriert sind, einen Strom zu erzeugen, der eingespeist wird, um unerwünschte Empfindungen zu reduzieren, sogar zu beseitigen, die von der Anregung durch den Stimulationsstrom stammen, **dadurch gekennzeichnet, dass** jede Stimulationselektrode (1, 2) von einer Stromeinspeiseelektrode (4, 11) umgeben ist, und dass eine Stimulationselektrode mindestens einen ersten Teil (1') enthält, der vollständig von einem ersten Teil einer Stromeinspeiseelektrode (4') umgeben ist, der selbst von einem zweiten Teil der Stimulationselektrode (1") vollständig umgeben ist, der selbst von einem zweiten Teil der Stromeinspeiseelektrode (4") umgeben ist.

2. System nach Anspruch 1, **dadurch gekennzeichnet, dass** die Konfiguration der Elektroden sich wiederholt.

## Claims

1. Electrode system for transcutaneous nerve and/or muscle stimulation, comprising:
- a pair of stimulation electrodes (1, 2) having a distance between them,
- a current generator (3) linked to the stimulation electrodes to generate a nerve and/or muscle stimulation current (9) between said stimulation electrodes (1, 2),
- a pair of current injection electrodes (4, 11) arranged at a distance from one of the stimulation electrodes which is less than the distance between the pair of stimulation electrodes (1, 2),
- an injection current generator (5, 12) linked to one of the stimulation electrodes (1, 2) and to one of the current injection electrodes,
the current injection electrodes (4, 11) and the corresponding current generator (5, 12) being configured to produce an injected current to reduce, even eliminate, undesirable sensations resulting from the excitation by said stimulation current, **characterized in that** each stimulation electrode (1, 2) is surrounded by a current injection electrode (4, 11) and **in that** a stimulation electrode comprises at least a first part (1') completely surrounded by a first part of the current injection electrode (4'), itself being completely surrounded by a second part of the stimulation electrode (1") which itself is completely surrounded by a second part of the current injection electrode (4").

2. System according to Claim 1, **characterized in that** the configuration of the electrodes is repeated.
